(19) Europäisches Patentamt
European Patent Office
Office européen des brevets

(11) Veröffentlichungsnummer: **0 039 810 A1**

(12) # EUROPÄISCHE PATENTANMELDUNG

(21) Anmeldenummer: **81103120.2**

(22) Anmeldetag: **25.04.81**

(51) Int. Cl.[3]: **C 07 C 85/11**
C 07 C 85/24, C 07 C 87/60

(30) Priorität: **08.05.80 DE 3017542**

(43) Veröffentlichungstag der Anmeldung:
**18.11.81 Patentblatt 81/46**

(84) Benannte Vertragsstaaten:
**CH DE FR GB LI**

(71) Anmelder: **BAYER AG**
**Zentralbereich Patente, Marken und Lizenzen**
**D-5090 Leverkusen 1, Bayerwerk(DE)**

(72) Erfinder: **Klauke, Erich, Dr.**
**Eichendorffweg 8**
**D-5068 Odenthal(DE)**

(72) Erfinder: **Braden, Rudolf, Dr.**
**Nothauser Feld 1**
**D-5068 Odenthal(DE)**

(54) **Verfahren zur Herstellung von 2-Trifluormethylanilin.**

(57) Die vorliegende Erfindung betrifft ein neues Verfahren zur Herstellung von 2-Trifluormethylanilin. Das Verfahren besteht in der reduktiven Entchlorierung von Verbindungen der Formel (I),

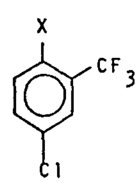

(1)

in welcher der Substituent X für die Nitro- oder die Aminogruppe steht. Die reduktive Entchlorierung erfolgt mit Wasserstoff in Gegenwart von Katalysatoren und von Säureakzeptoren.

EP 0 039 810 A1

Croydon Printing Company Ltd.

0039810

BAYER AKTIENGESELLSCHAFT
5090 Leverkusen, Bayerwerk
Zentralbereich
Patente, Marken und Lizenzen
Er/bc/c

## Verfahren zur Herstellung von 2-Trifluormethylanilin

Die vorliegende Erfindung betrifft ein neues Verfahren zur Herstellung von 2-Trifluormethylanilin. Das Verfahren besteht in der reduktiven Entchlorierung von Verbindungen der Formel (I), in welcher der Substituent X für die Nitro- oder die Aminogruppe steht. Die reduktive Entchlorierung erfolgt mit Wasserstoff in Gegenwart von Katalysatoren und von Säureakzeptoren.

Bislang wird 2-Trifluormethylanilin technisch als Nebenprodukt bei der Herstellung von 3-Trifluormethylanilin gewonnen. Bei der Nitrierung von Trifluormethylbenzol (andere Bezeichnung: Benzotrifluorid) wird ein Gemisch von Nitrotrifluormethylbenzolen gewonnen: 3-Nitrotrifluormethylbenzol in ca. 90 %iger Ausbeute der Theorie, 2-Nitrotrifluormethylbenzol in 8 bis 10 % Ausbeute und 4-Nitrotrifluormethylbenzol in 1 bis 2 % Ausbeute der Theorie. Die vorgenannten Nitrotrifluormethylbenzole werden anschließend zu den entsprechenden Aminen reduziert, dabei wird das 2-Trifluormethylanilin destillativ als Vorlaufprodukt abgetrennt. Somit ist die Verfügbarkeit des 2-Trifluormethylanilins an die Produktion seines Isomeren 3-Trifluormethylanilin gebunden. Diese Tatsache führt zu Schwierigkeiten in der

Versorgung mit 2-Trifluormethylanilin und es besteht daher ein dringendes Bedürfnis ein unabhängiges großtechnisches Verfahren zur selektiven Herstellung von 2-Trifluormethylanilin zur Verfügung zu haben.

Zur selektiven Herstellung des 2-Trifluormethylanilins sind bereits einige Verfahren bekannt geworden. So geht ein spezieller Weg vom 2-Acetaminotrifluormethylbenzol aus und verläuft nach dem folgenden Formelschema:

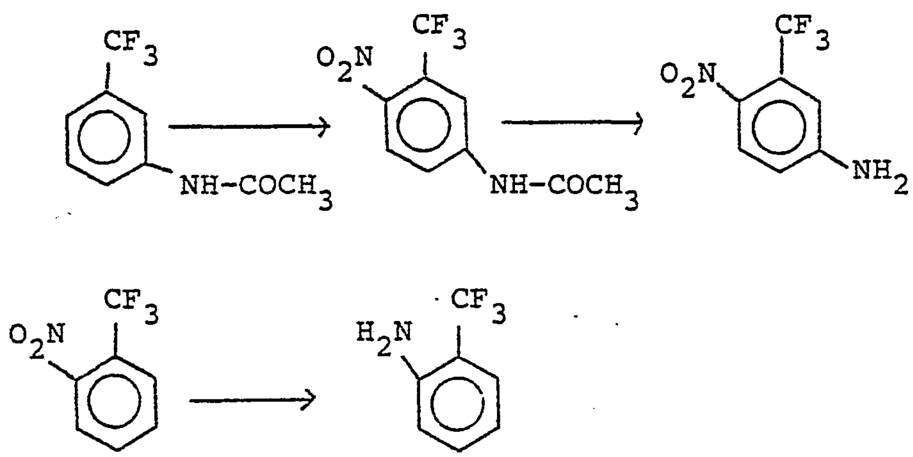

(s. Tetrahedron 8, 67 (1960))

Ein anderer Weg zum 2-Trifluormethylanilin geht vom 2-Methylbenzonitril aus, wobei die letztgenannte Verbindung nach einer Seitenkettenchlorierung und anschließender Fluorierung mit $SbF_3$ zum Säureamid hydrolysiert und anschließend durch Hoffmann-Abbau in das 2-Trifluormethylanilin übergeführt wird (Literatur: Z. obsc. chim. 23, 988 (1953)).

Einen weiteren Zugang zum 2-Trifluormethylanilin erhält man, indem man in o-Toluidin die Aminogruppe durch Acylierung mit Phthalsäure schützt, dann anschließend die $CH_3$-Seitenkette chloriert und schließlich mit HF fluoriert. Durch abschließende Hydrolyse der Acylschutzgruppe erhält man das 2-Trifluormethylanilin.

Allen bisher genannten Methoden zur selektiven Herstellung des 2-Trifluormethylanilins haften die Nachteile von Vielstufensynthesen an oder es wird wie im letztgenannten Verfahren die Verwendung von großballastigen Schutzgruppen erforderlich, welche eine eigene Kreisführung notwendig machen.

Es wurde nun überraschend gefunden, daß man auf eine elegante und einfache Weise ausgehend von einem großtechnisch leicht zugänglichen Produkt zum gewünschten 2-Trifluormethylanilin gelangen kann. In Gegenwart von Hydrierkatalysatoren und Säureakzeptoren gelingt es, das Chloratom in 2-Amino-5-chlor-trifluormethylbenzol sowie in 2-Nitro-5-chlor-trifluormethylbenzol mit Wasserstoff zu entfernen, ohne daß die im Molekül vorhandenen Trifluormethylseitenketten angegriffen werden. Dies ist überraschend, da aus der Literatur bekannt ist daß Trifluormethylseitenketten am Benzolkern durch o-ständige Aminogruppen stark destabilisiert werden (J. Am. Soc. 76 (1956) 1051, J. Chem. Soc. 1954, 3846).

Das als Ausgangsprodukt einsetzbare 2-Amino-5-chlor-trifluormethylbenzol ist ein bekanntes Farbstoffzwischenprodukt, das auch unter dem Namen Echtscharlach VD-Base bekannt ist. 2-Amino-5-chlor-trifluormethylbenzol wird

technisch aus dem 2-Nitro-5-chlor-trifluormethylbenzol durch katalytische Hydrierung hergestellt. Aus diesem Grunde kann man beide Hydrierungen zusammenfassen, d.h. als Ausgangsmaterial für die erfindungsgemäße Herstellung von 2-Amino-trifluormethylbenzol kann sowohl das 2-Amino-5-chlor-trifluormethylbenzol als auch das 2-Nitro-5-chlor-trifluormethylbenzol eingesetzt werden:

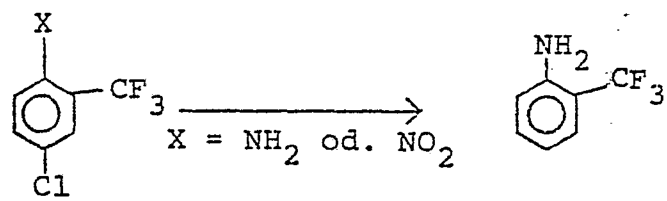


Die Hydrierung wird im allgemeinen so durchgeführt, daß man im Autoklaven 2-Amino-5-chlor-trifluormethylbenzol bzw. 2-Nitro-5-chlor-trifluormethylbenzol in einem Lösungsmittel vorlegt, den Säureakzeptor und den Katalysator zugibt, Wasserstoff hinzugibt, unter Rühren aufheizt und bei der Reaktionstemperatur bis zum Ende der Wasserstoffaufnahme durch das Reaktionsgemisch Wasserstoff unter Druck einleitet.

Die Hydrierung kann sowohl im neutralen als auch im basischen Medium durchgeführt werden. Die Art des zu verwendenden Säureakzeptors ist beim erfindungsgemäßen Verfahren nicht kritisch. Geeignete Säureakzeptoren sind beispielsweise Alkali- und Erdalkalihydroxide, -oxide, -carbonate oder -acetate wie z.B. Natriumhydroxid, Natriumacetat, Natriumcarbonat, Kaliumhydroxid, Kaliumcarbonat, Lithiumcarbonat, Calciumoxid, Magnesiumoxid, Ammoniak und Amine, insbesondere tertiäre Amine, wie z.B. Trimethylamin, Triethylamin, Pyridin und Picolin. Es ist beim erfindungsgemäßen Verfahren auch

möglich, basische Ionenaustauscher als Säureakzeptoren zu verwenden. In der Regel soll wenigstens ein Mol Säureakzeptor pro Mol herzustellenden 2-Trifluormethylanilins eingesetzt werden. Es kann aber auch vorteilhaft sein, tertiäre Amine im Überschuß als Lösungsmittel zu verwenden. In einer besonderen Ausführungsform des erfindungsgemäßen Verfahrens können der Säureakzeptor, der Katalysator und gegebenenfalls das Lösungsmittel im Hydrierreaktor vorgelegt und das 2-Trifluormethyl-4-chloranilin bzw. das 2-Trifluormethyl-4-chlornitrobenzol zudosiert werden. Die Hydrierung kann in einem gegenüber den Reaktionspartnern beim erfindungsgemäßen Verfahren inerten Lösungsmittel durchgeführt werden. Inerte Lösungsmittel sind beispielsweise Alkohole mit vorzugsweise 1 bis 6 Kohlenstoffatomen wie z.B. Methanol, Ethanol, Isopropanol, n-Propanol, Isobutanol, Butanol-2, Ethylenglykol. Weiterhin können als inerte Lösungsmittel beispielsweise auch Ether wie Glykolmethylether, Glykoldimethylether, Tetrahydrofuran, Dioxan, Anisol, sowie Kohlenwasserstoffe wie z.B. Cyclohexan, Methylcyclohexan, Toluol, Xylol aber auch andere Lösungsmittel wie z.B. Essigsäure sowie Wasser eingesetzt werden. Es ist beim erfindungsgemäßen Verfahren weiterhin auch möglich, das Endprodukt 2-Trifluormethylanilin als Lösungsmittel einzusetzen. Die beim erfindungsgemäßen Verfahren einsetzbaren Lösungsmittel können auch als Gemische verwendet werden. Beim erfindungsgemäßen Verfahren besonders bevorzugte Lösungsmittel sind Methanol und Ethanol.

Die Konzentration der chlorhaltigen Ausgangsverbindung (2-Amino-5-chlor-trifluormethylbenzol bzw. 2-Nitro-5-chlor-trifluormethylbenzol) im Lösungsmittel kann

weit variiert werden. Bevorzugt werden im allgemeinen Konzentrationen von wenigstens 25 Gew.-% (bezogen auf das eingesetzte Lösungsmittel) der vorgenannten Ausgangsverbindungen.

Die erfindungsgemäße Reaktion wird bei Temperaturen von 15 bis 200°C durchgeführt, vorzugsweise arbeitet man bei 30 bis 150°C. Die verwendete Reaktionstemperatur richtet sich insbesondere nach der Aktivität des beim erfindungsgemäßen Verfahren eingesetzten Hydrierkatalysators.

Die Hydrierung kann bei Normaldruck oder auch bei erhöhtem Druck durchgeführt werden. Die bevorzugte Arbeitsweise für das erfindungsgemäße Verfahren ist die bei erhöhtem Druck. Als erhöhter Druck sei beispielsweise ein Druck von 5 bis 250 bar, bevorzugt 20 bis 100 bar, genannt.

Das erfindungsgemäße Verfahren kann sowohl in der Rieselphase als auch in der Flüssigphase durchgeführt werden. Die bevorzugte Durchführung ist die in der Flüssigphase. Besonders bevorzugt ist die Durchführung in der Flüssigphase bei erhöhtem Druck. Das erfindungsgemäße Verfahren kann sowohl diskontinuierlich als auch kontinuierlich durchgeführt werden. Das erfindungsgemäße Verfahren wird in Gegenwart eines Katalysators durchgeführt, der für die Katalyse von Hydrierungen eingesetzt werden kann und im weiteren Hydrierungskatalysator genannt wird. Als Hydrierungskatalysator sei beispielsweise ein solcher genannt, der mindestens ein Metall der 8. Gruppe des Periodensystems (Mendelejew) und/oder Kupfer oder mindestens eines dieser Metalle

in Kombination mit mindestens einem Metall aus der Gruppe Vanadium, Chrom und Mangan in metallischer und/ oder oxidischer Form enthält. Der Hydrierungskatalysator kann zusammen mit einem inerten Trägermaterial oder auch ohne Trägermaterial zur Anwendung gelangen. Beispiele für inerte Trägermaterialien sind synthetische und natürlich vorkommende, gegebenenfalls physikalisch oder chemisch veränderte Stoffe wie Aluminiumoxide, Kieselsäure, Kieselgur, Silikate, Aluminiumsilikate, Montmorillonit, Zeolithe, Spinnelle, Kaolin, Ton, Magnesiumsilikat, Asbest, Bimsstein, Dolomit, Erdalkalicarbonat, Erdalkalisulfate, Zinkoxid, Zirkonoxid, Siliciumcarbid, Borphosphat, Aluminiumphosphat, Aktivkohle und als organische Katalysatorträger natürlich vorkommende, physikalisch oder chemisch veränderte oder synthetische Verbindungen mit hohem Molgewicht wie Seide, Polyamide, Polystyrole, Zellstoff oder Polyurethane, wobei die Träger z.B. in Form von Kugeln, Strängen, Fäden, Zylindern, Polygonen oder in Pulverform vorliegen können.

Die vorstehend genannten Trägerkatalysatoren enthalten im allgemeinen etwa 1 bis 70, vorzugsweise 5 bis 65 Gew.-% des katalytisch aktiven Metalls bezogen auf die Gesamtmasse des Trägerkatalysators. Die katalytisch aktiven Metalle können homogen in Trägermaterial verteilt oder in der äußeren Schicht oder auf der Oberfläche des Trägers gelagert sein.

Die Katalysatoren können ferner einen oder mehrere Beschleuniger oder Aktivatoren wie Lithium, Natrium, Kalium, Calcium, Barium, Silber, Gold, Beryllium, Lanthan, Cer, Vanadium, Niob, Tantal, Molybdän oder

Wolfram in Mengen bis zu 10 Gew.-%, bevorzugt in Mengen bis zu 1 Gew.-%, enthalten.

Als aktive Stoffe in metallischer oder oxidischer Form für die erfindungsgemäß einsetzbaren Hydrierungskatalysatoren seien beispielsweise genannt: Palladium, Platin, Nickel, Eisen oder Kupfer. Die genannten Metalle können sowohl einzeln als auch im Gemisch mehrerer im Hydrierungskatalysator enthalten sein. Sie können ferner mit einem Metall aus der Gruppe Vanadium, Chrom und Mangan kombiniert sein. Bevorzugte Hydrierungskatalysatoren für das erfindungsgemäße Verfahren sind solche, die Nickel und/oder Palladium in metallischer oder oxidischer Form allein oder in Kombination mit mindestens einem der genannten Metalle enthalten, beispielsweise Katalysatoren vom Raney-Typ, wie Raney-Nickel, Raney-Nickel-Eisen, Raney-Nickel-Kobalt, Raney-Nickel-Kupfer, durch Reduktion von Nickelsalzen mit Zinkstaub, Alkalihydrid, Boranaten, Borwasserstoff, Metallalkylverbindungen oder Hydrazin hergestelltes metallisches Nickel, wie Urushibara-Nickel, durch Reduktion von Nickeloxid oder Gemischen aus Nickeloxid und mindestens einem weiteren Metalloxid mit Wasserstoff hergestellte metallische Katalysatoren, Nickeloxid oder Gemische aus Nickeloxid mit mindestens einem weiteren Metalloxid, wie Nickeloxid-chromoxid, Nickeloxidmanganoxid-, Kupferoxid-, Chromoxid-, Kupferoxid- oder Trägerkatalysatoren, wie Nickel-auf-Kieselgur, Nickel-auf-Aluminiumoxid, Nickel-Kupfer auf Aluminiumoxid oder Nickel-Mangan auf Aluminiumoxid oder Palladium-Katalysatoren, wie beispielsweise Palladium-auf-Kohle, Palladium-auf-Kieselgur, Palladium-auf-Calciumcarbonat oder Palladium-auf-Tonerde.

Besonders bevorzugte Katalysatoren sind Raney-Nickel mit 90 Gew.-% Nickel und weniger als 1 Gew.-% Eisen, Calcium und Natrium, Raney-Nickel-Eisen mit 5 bis 30 Gew.-% Eisen und weniger als 1 Gew.-% Calcium und Natrium; Palladium-auf-Kohle mit 0,5 bis 5 Gew.-% Palladium.

Die Reaktionszeit kann beim erfindungsgemäßen Verfahren bis zu 10 Stunden betragen. In der Regel werden Temperatur und Katalysator bei einem entsprechenden Wasserstoffdruck so gewählt, daß die Hydrierung in ungefähr 5 Stunden beendet ist. Aus wirtschaftlichen Gründen kann es zweckmäßig sein, daß beispielsweise an einem Palladiumkatalysator mit einer Alkaliverbindung als Säureakzeptor bei Temperaturen von 30 bis 150°C in weniger als 1 Stunde ein praktisch vollständiger Umsatz der chlorhaltigen Ausgangsverbindung erreicht wird.

Beim erfindungsgemäßen Verfahren wird abschließend nach dem Abkühlen und Entspannen des Autoklaven der Ansatz filtriert, der Filterrückstand zweimal mit dem eingesetzten Lösungsmittel gewaschen und das Filtrat schließlich destilliert. Man erhält das gewünschte 2-Trifluormethylanilin in hohen Ausbeuten.

Beispiel 1

In einem 0,7 l Autoklaven werden 195,5 g 2-Trifluormethyl-4-chloranilin in 200 ml Methanol, 100 g Natriumcarbonat und 10 g Raney-Nickel vorgelegt. Nach dem Spülen des Autoklaven mit Wasserstoff wird bei 60°C unter Steigerung der Temperatur bis auf 100°C mit einem Wasserstoffdruck von 50 bar hydriert. Nach 5 Stunden ist die Hydrierung beendet. Man läßt abkühlen und den Autoklaven entspannen. Nach Filtration des Autoklaveninhalts und zweimaliger Wäsche des Filterkuchens mit je 150 ml Methanol wird das Filtrat anschließend destilliert. Der Vorlauf besteht aus Methanol, der Hauptlauf von 136 g destilliert bei 63 bis 66°C bei einem Druck von 20 mbar und hat eine Reinheit von 97 % 2-Trifluormethylanilin, $n_D^{20}$ : 1,4800.

Aus dem Filterkuchen lassen sich durch Versetzen mit Wasser und Abtrennen der organischen Phase noch 9 g 2-Trifluormethylanilin isolieren. Der Umsatz beträgt 96 % und die Ausbeute 88 % der Theorie.

Beispiel 2

Apparatur und Reaktionsbedingungen erfolgen hier in Analogie zu Beispiel 1. Es werden im vorliegenden Fall 195,5 g 2-Amino-5-chlor-trifluormethylbenzol, 120 g Triethylamin, 200 ml Methanol und 30 g Raney-Nickel eingesetzt. Man arbeitet bei einer Reaktionstemperatur von 140°C, der Wasserstoffdruck beträgt 50 bar. Die Aufarbeitung erfolgt wie beim Beispiel 1 beschrieben durch Destillation.
Ausbeute: 147 g 2-Trifluormethylanilin, d.h. 89 % der Theorie.

Beispiel 3

In einen 1,3 l Autoklaven werden 150 g 2-Nitro-5-chlor-trifluormethylbenzol, 1,50 g Triethylamin, 20 g Raney-Nickel und 700 ml Methanol eingefüllt. Der Autoklav wird verschlossen, zweimal mit Wasserstoff gespült und dann werden 60 bar molekularer Wasserstoff aufgedrückt. Unter Rühren heizt man bis auf 120°C und hält durch Nachdrücken von Wasserstoff den Druck weiterhin auf 60 bar. Nach 5 Stunden ist die Wasserstoffaufnahme beendet. Man läßt abkühlen, entspannt und filtriert anschließend. Das Filtrat wird destilliert. Im Vorlauf werden Methanol, überschüssiges Triethylamin sowie Wasser abgetrennt. Dann destillieren bei einem Siedepunkt von 65 bis 67°C (Druck: 15 mbar) 88 g 2-Trifluormethylanilin ($n_D^{20}$ : 1,4802) über. Aus dem Rückstand von 110 g lassen sich nach dem Einstellen des pH-Wertes auf einen Wert über pH 7 Triethylamin und 16 g eines Gemisches bestehend aus etwa gleichen Teilen 2-Amino-trifluormethylbenzol und 2-Amino-5-chlor-trifluormethylbenzol isolieren.

Beispiel 4

50 g 2-Nitro-5-chlor-trifluor-methylbenzol werden in 250 ml Methanol in Gegenwart von 27 g Triethylamin und 5 g Palladium-Kohle (5 Gew.-% Pd-Gehalt) gelöst und in einem Rührautoklaven bei 50°C und 30 bar Wasserstoffdruck 3 Stunden hydriert. Das Reaktionsgemisch wird filtriert. Die Hauptmenge des Lösungsmittels wird bei Normaldruck abdestilliert und der Rückstand mit Toluol versetzt. Toluolphase wird abgetrennt und getrocknet.

Die Destillation ergibt 27,9 g 2-Trifluormethylanilin $Kp_{15\ mbar}$ 61-63°C, $n_D^{20}$ 1,4819, das sind 78 % der Theorie.

## Patentansprüche

1) Verfahren zur Herstellung von 2-Trifluormethylanilin, dadurch gekennzeichnet, daß man eine Verbindung der Formel

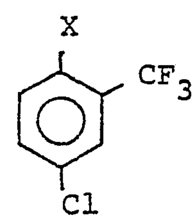

(I)

in welcher
X für $NO_2$ oder $NH_2$ steht,

in Gegenwart eines Säureakzeptors und eines Hydrierkatalysators bei 15 bis 200°C hydriert.

2) Verfahren nach Anspruch 1, dadurch gekennzeichnet, daß man bei einer Temperatur von 30 bis 150°C und bei 5 bis 250 bar Wasserstoffdruck hydriert.

3) Verfahren nach Anspruch 1 oder 2, dadurch gekennzeichnet, daß man als Hydrierkatalysator einen Nickel-, Palladium-, Platin-Eisen- und/oder Kupferhaltigen Katalysator verwendet.

4) Verfahren nach den Ansprüchen 1 bis 3, dadurch gekennzeichnet, daß man einen Palladium-haltigen Katalysator und/oder Raney-Nickel als Katalysator einsetzt.

Europäisches Patentamt

# EUROPÄISCHER RECHERCHENBERICHT

0039810

Nummer der Anmeldung

EP 81 10 3120

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| X | LU - A - 62 645 (BAYER)<br>* Ansprüche *<br>-- | 1-4 | C 07 C 85/11<br>85/24<br>87/60 |
| X | FR - A - 2 285 355 (I.C.I.)<br>* Ansprüche; Beispiel 2 *<br>& DE - A - 2 540 740<br>-- | 1-4 | |
| X | US - A - 4 140 719 (R.J. TULL u.a.)<br>* Ansprüche; Spalten 1,2 *<br>-- | 1-4 | RECHERCHIERTE SACHGEBIETE (Int. Cl.³) |
| | FR - A - 2 298 531 (BAYER)<br>* Ansprüche *<br>-- | 1 | C 07 C 85/11<br>85/24<br>87/60 |
| | US - A - 4 172 095 (M. STEINMAN und Y.S. WONG)<br>* Ansprüche; Beispiel 2 *<br>---- | 1 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 03-08-1981 | MOREAU |